# EUROPEAN PATENT SPECIFICATION

(11) **EP 4 598 468 B1**
(45) Date of publication and mention of the grant of the patent: **25.11.2026**
(21) Application number: 23789761.6
(22) Date of filing: 05.10.2023
(51) Int. Cl.: A61B 34/10, A61F 2/24, A61B 17/00

(54) **COMPUTER IMPLEMENTED METHOD FOR ASSESSING VALVE-IN-VALVE RISKS**
COMPUTERIMPLEMENTIERTES VERFAHREN ZUR BEURTEILUNG VON VENTIL-IN-VENTIL-RISIKEN
PROCÉDÉ MIS EN OEUVRE PAR ORDINATEUR D'ÉVALUATION DE RISQUES ASSOCIÉS À L'INTERVENTION DE VALVE-DANS-VALVE

(30) Priority: 07.10.2022 US 202263414082 P
(43) Date of publication of application: 13.08.2025
(60) Divisional application: 26205897.7 / 4 834 240
(73) Proprietor: Medtronic, Inc., Minneapolis, MN 55432 (US)
(72) Inventor: SPENCER, Julianne H., Minneapolis, Minnesota 55432 (US); TADROS, Hatem, Minneapolis, Minnesota 55432 (US); GARDE, Kshitija P., Minneapolis, Minnesota 55432 (US); POPMA, Jeffrey J., Minneapolis, Minnesota 55432 (US)
(74) Representative: Zimmermann & Partner Patentanwälte mbB
(86) International application number: PCT/IB2023/060025
(87) International publication number: WO 2024/075063

(56) References cited:
- US-A- 5 957 949
- OCHIAI TOMOKI ET AL: "Risk of Coronary Obstruction Due to Sinus Sequestration in Redo Transcatheter Aortic Valve Replacement", JACC: CARDIOVASCULAR INTERVENTIONS, ELSEVIER, AMSTERDAM, NL, vol. 13, no. 22, 16 November 2020 (2020-11-16), pages 2617 - 2627, XP086345448, ISSN: 1936-8798, [retrieved on 20201116], DOI: 10.1016/J.JCIN.2020.09.022
- TARANTINI GIUSEPPE ET AL: "Transcatheter Aortic Valve Replacement in Failed Transcatheter Bioprosthetic Valves", JACC: CARDIOVASCULAR INTERVENTIONS, ELSEVIER, AMSTERDAM, NL, vol. 15, no. 18, 19 September 2022 (2022-09-19), pages 1777 - 1793, XP087183190, ISSN: 1936-8798, [retrieved on 20220919], DOI: 10.1016/J.JCIN.2022.07.035
- HAMID NADIRA B ET AL: "Transcatheter Valve Implantation in Failed Surgically Inserted Bioprosthesis Review and Practical Guide to Echocardiographic Imaging in Valve-in-Valve Procedures", JACC: CARDIOVASCULAR IMAGING, ELSEVIER, AMSTERDAM, NL, vol. 8, no. 8, 10 August 2015 (2015-08-10), pages 960 - 979, XP029258705, ISSN: 1936-878X, DOI: 10.1016/J.JCMG.2015.01.024

## Description

### FIELD

The present disclosure relates to systems and methods for assessing or predicting potential concerns associated with proposed valve-in-valve procedure. More particularly, it relates to systems and methods for assessing or predicting risks to a patient under consideration for receiving a replacement transcatheter aortic valve within a first or initial bioprosthetic aortic valve.

### BACKGROUND

A human heart includes four heart valves that determine the pathway of blood flow through the heart: the mitral valve, the tricuspid valve, the aortic valve, and the pulmonary valve. The mitral and tricuspid valves are atrio-ventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Diseased or otherwise deficient heart valves can be repaired or replaced using a variety of different types of heart valve surgeries. One conventional technique involves an open-heart surgical approach that is conducted under general anesthesia, during which the heart is stopped and blood flow is controlled by a heart-lung bypass machine.

More recently, minimally invasive approaches have been developed to facilitate catheter-based implantation of the valve prosthesis on the beating heart, intending to obviate the need for the use of classical sternotomy and cardiopulmonary bypass. In general terms, an expandable prosthetic valve is compressed about or within a catheter, inserted inside a body lumen of the patient, such as the femoral artery, and delivered to a desired location in the heart.

The heart valve prosthesis employed with catheter-based, or transcatheter, procedures generally includes an expandable multi-level frame or stent that supports a valve structure having a plurality of leaflets. The frame can be contracted during percutaneous transluminal delivery, and expanded upon deployment at or within the native valve. One type of valve stent can be initially provided in an expanded or uncrimped condition, then crimped or compressed about a balloon portion of a catheter. The balloon is subsequently inflated to expand and deploy the prosthetic heart valve. With other stented prosthetic heart valve designs, the stent frame is formed to be self-expanding. With these systems, the valved stent is crimped down to a desired size and held in that compressed state within a sheath for transluminal delivery. Retracting the sheath from this valved stent allows the stent to self-expand to a larger diameter, fixating at the native valve site. In more general terms, then, once the prosthetic valve is positioned at the treatment site, for instance within an incompetent native valve, the stent frame structure may be expanded to hold the prosthetic valve firmly in place. One example of a stented prosthetic valve is disclosed in U.S. Pat. No. 5,957,949 to Leonhardt et al.

In recent years, an increasing number of prosthetic heart valves have been implanted, and in the near future more and more patients will be candidates for reoperation due, for example, to changes in anatomy, structural deterioration, etc. Valve-in-valve transcatheter valve replacement ("TAV-in-TAV") has become a safe and effective alternative to surgery under these and other circumstances.

Patient screening for a valve-in-valve transcatheter prosthetic aortic heart valve can be challenging due to the anatomical complexities of the patient population. Some screening processes may be costly, time-consuming, subjective, and not sufficiently predictive. For example, some screening processes may not sufficiently evaluate or predict a risk of coronary sequestration and/or access challenges. The leaflets of the previously-implanted prosthetic valve are displaced to create a cylinder effect (or "neo-skirt") causing sinus sequestration and sealing off flow to the coronaries.

The present disclosure addresses problems and limitations associated with the related art.

Ochiai Tomoki et al describes the "Risk of Coronary Obstruction Due to Sinus Sequestration in Redo Transcatheter Aortic Valve Replacement", JACC: Cardiovascular Interventions, Elsevier, Amsterdam, NL, vol. 13, no. 22, 16 November 2020, pages 2617-2627, XP086345448.

Tarantini Giuseppe et al describes "Transcatheter Aortic Valve Replacement in Failed Transcatheter Bioprosthetic Valves", JACC: Cardiovascular Interventions, Elsevier, Amsterdam, NL, vol. 15, no. 18, 19 September 2022, pages 1777-1793, XP087183190.

### SUMMARY

Some aspects of the present disclosure are directed to methods for evaluating a proposed valve-in-valve procedure in which a replacement transcatheter aortic valve will be deployed within a first or initial bioprosthetic aortic valve. The methods include selecting predetermined benchmark measurements of a valve-in-valve combination comprising a known transcatheter aortic valve deployed within a known bioprosthetic aortic valve. Images of anatomy of the patient are received. Anatomical measurements of the first bioprosthetic aortic valve relative to the anatomy are obtained from the received images. The predetermined benchmark measurements and the anatomical measurements are reviewed. The risks of a valve-in-valve procedure to the patient are evaluated based, at least in part, upon the review. In some embodiments, the methods of the present disclosure consider risks of sinus sequestration and/or coronary artery access obstructions presented by a proposed transcatheter aortic valve-in-transcatheter aortic valve ("TAV-in-TAV") procedure. In some embodiments, the predetermined benchmark measurements include a height of a neo-skirt of the combination valve-in-valve. In some embodiments, reviewing the predetermined benchmark measurements and the anatomical measurements include one or more of: comparing a neo-skirt height value of the predetermined benchmark measurements with a coronary artery ostium height value of the anatomical measurements; comparing a neo-skirt height value of the predetermined benchmark measurements with a sinotubular junction height of the anatomical measurements; assessing a residual area or volume or distance between the first bioprosthetic aortic valve and native anatomy at a level of a native sinotubular junction; and assessing a residual area or volume or distance between the first bioprosthetic aortic valve and native anatomy at a level corresponding with a neo-skirt height value of the predetermined benchmark measurements. In some embodiments, the evaluation is performed for a patient who has previously received a bioprosthetic aortic valve and is under consideration for receiving a candidate replacement transcatheter aortic valve; with these and related embodiments, the step of evaluating includes determining whether the candidate replacement transcatheter aortic valve is appropriate for the patient. In other embodiments, the evaluation is performed for a patient who has not previously received a bioprosthetic aortic valve and is under consideration for receiving a candidate initial bioprosthetic aortic valve. With these and related embodiments, the step of evaluating includes determining whether the candidate initial bioprosthetic aortic valve is appropriate for the patient; the baseline assessment can also include assumptions of how the initial bioprosthetic valve will be implanted, such as depth of implant and centering of the valve within the sinus vessel.

### BRIEF DESCRIPTION OF DRAWINGS

FIG. 1 is a schematic sectional illustration of a mammalian heart having native valve structures;
FIG. 2 is a schematic sectional illustration of a native aortic valve and surrounding anatomy;
FIG. 3 is a schematic sectional illustration of a bioprosthetic aortic valve implanted to a native aortic valve annulus;
FIG. 4A is a simplified side sectional view of a first bioprosthetic aortic valve poised for placement within a second bioprosthetic aortic valve;
FIG. 4B is a simplified side sectional view of the bioprosthetic aortic valves of FIG. 4A upon final deployment of the first bioprosthetic aortic valve within the second bioprosthetic aortic valve;
FIG. 5A is a schematic sectional illustration of a valve-in-valve arrangement at a native aortic valve;
FIG. 5B is a schematic sectional illustration of another valve-in-valve arrangement at a native aortic valve along with a surgical device;
FIG. 6 is a block diagram illustrating a computing system for evaluating a patient having indications for a transcatheter bioprosthetic aortic valve replacement procedure;
FIG. 7 is a flow diagram illustrating a method of evaluating a patient having indications for a transcatheter aortic valve replacement procedure;
FIG. 8 is a block diagram of a library of predetermined valve-in-valve benchmark measurement data useful with the systems and methods of the present disclosure;
FIG. 9A is a simplified side sectional view of a valve-in-valve combination and identifying benchmark measurements data useful with the systems and methods of the present disclosure;
FIG. 9B is a simplified side sectional view of another valve-in-valve combination and identifying benchmark measurements data useful with the systems and methods of the present disclosure;
FIG. 10 is an example table of benchmark measurements useful with the systems and methods of the present disclosure;
FIG. 11 is a schematic sectional illustration of a bioprosthetic aortic valve implanted to native anatomy and identifying anatomical measurements useful with the systems and methods of the present disclosure;
FIG. 12 is a flow diagram illustrating a method for evaluating a risk of sinus sequestration in accordance with principles of the present disclosure and useful with the method of FIG. 7;
FIG. 13 is a flow diagram illustrating another method for evaluating a risk of sinus sequestration in accordance with principles of the present disclosure and useful with the method of FIG. 7; and
FIG. 14 is a flow diagram illustrating a method for evaluating a risk of coronary artery access obstruction in accordance with principles of the present disclosure and useful with the method of FIG. 7.

### DETAILED DESCRIPTION

Specific embodiments of the present disclosure are now described with reference to the figures, wherein like reference numbers indicate identical or functionally similar elements.

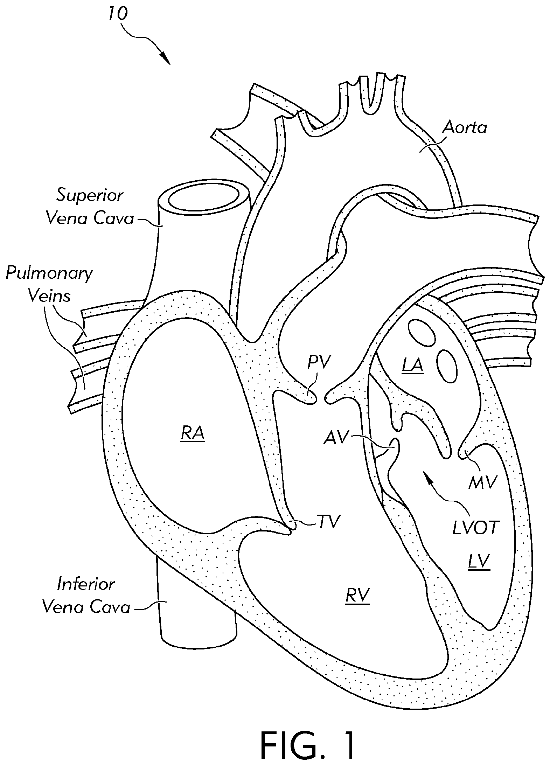

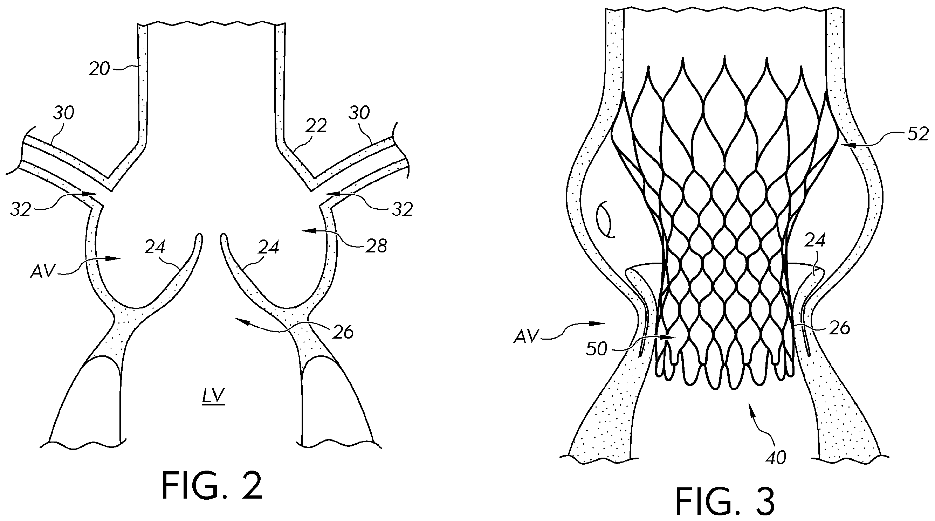

FIG. 1 is a schematic sectional illustration of a mammalian heart 10 that depicts the four heart chambers (right atria RA, right ventricle RV, left atria LA, left ventricle LV) and native valve structures (tricuspid valve TV, mitral valve MV, pulmonary valve PV, aortic valve AV). FIG. 2 is a schematic sectional illustration of the aortic valve AV and surrounding anatomy. Referring to FIGS. 1 and 2 together, the heart 10 comprises the left atrium LA that receives oxygenated blood from the lungs via the pulmonary veins. The left atrium LA pumps the oxygenated blood through the mitral valve MV and into the left ventricle LV during ventricular diastole. The left ventricle LV contracts during systole and blood flows outwardly through the aortic valve AV, into the aorta and to the remainder of the body.

Patient anatomy at and adjacent the aortic valve AV include an aorta 20, sinotubular junction ("STJ") 22, native leaflets 24, aortic valve annulus 26, sinus region (or Sinus of Valsalva) 28, coronary arteries 30 each having a coronary ostium 32, and the left ventricle LV. Defects or disease (e.g., aortic valve stenosis) can prevent the aortic valve AV from opening correctly, reducing blood flow from the heart to the patient's body. Under these and other circumstances, a defective aortic valve can be replaced or repaired by a bioprosthetic aortic valve. Some bioprosthetic aortic valve are intended to be surgically implanted, while others are configured for placement on a minimally invasive basis. For example, a transcatheter aortic valve (or "TAV") is installed to the patient via a transcatheter aortic valve replacement ("TAVR") procedure. The transcatheter aortic valve replacement procedure is also sometimes referred to as transcatheter aortic valve implantation ("TAVI"). FIG. 3 illustrates, in simplified form, one example of a bioprosthetic aortic valve 40 deployed to the native aortic valve AV. With the non-limiting example of FIG. 3, the bioprosthetic aortic valve 40 is a transcatheter aortic valve and generally includes a valve structure 50 (referenced generally) supported by a stent or stent frame 52. The stent frame 52 secures the bioprosthetic valve 50 to the native annulus 26. With some techniques, the native leaflets 24 are left in place, but are spaced or held away from (and thus do not interfere with) the valve structure 50 by the stent frame 52. In other instances, some or all of the native leaflets 24 can be removed.

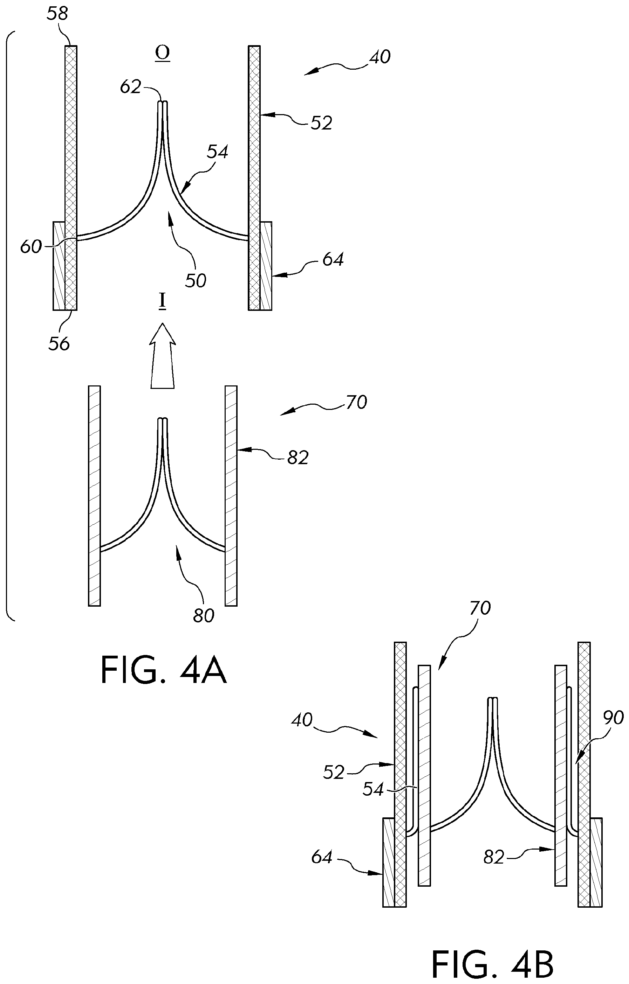

Regardless of the type or design, over time the bioprosthetic valve 40 may deteriorate and/or may no longer be optimal for the patient's changing anatomy (e.g., where the bioprosthetic valve 40 was implanted to a younger patient). A potential, viable approach to address these and other concerns is deployment of a second, or replacement, transcatheter aortic valve within the previously-implanted bioprosthetic valve 40 (also known as "valve-in-valve"). As a point of reference, a simplified representation of the previously-implanted bioprosthetic valve 40 is shown in FIG. 4A, along with a second or replacement transcatheter valve 70. As mentioned above, the previously-implanted bioprosthetic valve 40 includes the stent frame 52 maintaining the valve structure 50 that otherwise includes or provides two or more leaflets 54. An arrangement of the leaflets 54 relative to the stent frame 52 generates an inflow side I opposite an outflow side O. With this flow direction in mind, the stent frame 52 extends between a first or inflow end 56 and a second or outflow end 58. The leaflets 54 are secured relative to the stent frame 52 at a base 60, and extend from the stent frame 52 to a free margin 62. A skirt material 64 is typically provided along the stent frame 52 that extends from the inflow end 56. The replacement transcatheter valve 70 can have various designs, and generally includes a valve structure 80 supported by a stent or stent frame 82. As part of a valve-in-valve procedure, the replacement transcatheter valve 70 is deployed within the previously-implanted bioprosthetic valve 40 as generally reflected by FIG. 4B. Upon final deployment, the stent frame 82 of the replacement transcatheter valve 70 pins a portion or an entirety of the leaflets 54 of the previously-implanted bioprosthetic valve 40 to the stent frame 52 of the previously-implanted bioprosthetic valve 40. Because the previously-implanted bioprosthetic valve 50 and the replacement transcatheter valve 70 may not have the same design or footprint and/or due to variations in a location of the replacement transcatheter valve 70 relative to the previously-implanted bioprosthetic valve 40, the leaflets 54 may be partially or fully pinned between the stent frames 52, 82. Regardless, the pinned leaflets 54 combine with the skirt 64 to effectively create a barrier, or "neo-skirt", along the stent frame 82 of the replacement transcatheter valve 70. For ease of understanding, the neo-skirt is generally labeled as "90" in FIG. 4B. The new neo-skirt 90 is typically defined from the inflow end 56 and includes the skirt 64 of the outer (or previously-implanted) valve 40.

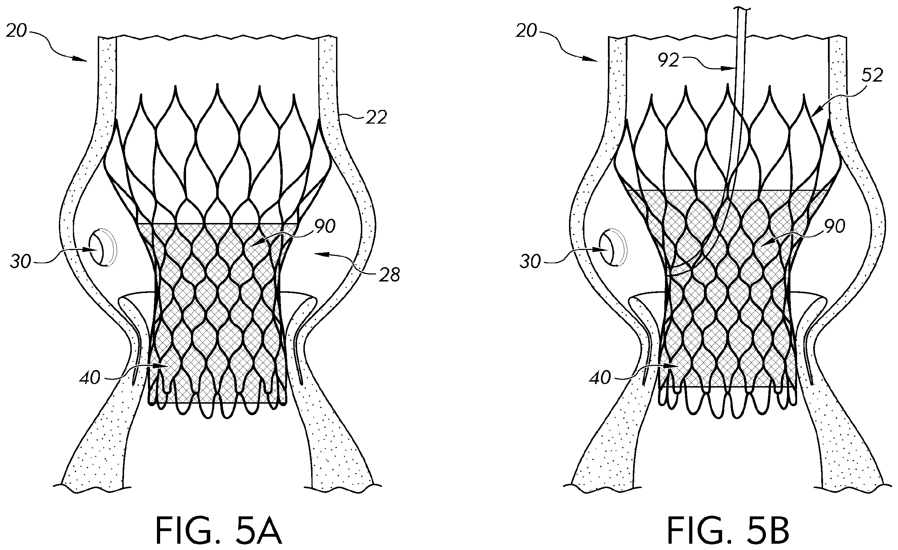

While viable for many patients, the valve-in-valve procedure may give rise to certain concerns. For example, shading in FIG. 5A generally reflects a possible arrangement of the neo-skirt 90 (e.g., the pinned leaflets) following deployment of the replacement transcatheter valve 70 (hidden in FIG. 5A). Under some circumstances, for examples where the neo-skirt 90 extends to or above the STJ 22, the neo-skirt 90 may act to partially or completely isolate or "sequester" the coronary sinus 28 from the aorta 20, thus partially or completely obstructing blood flow to the coronary arteries (an ostium 30 of one of the coronary arteries is labeled in FIG. 5A). Alternatively or in addition, even if coronary perfusion is maintained around the nested valves 40, 70, the neo-skirt 90 may render accessing one or more of the coronary ostia 30 exceedingly challenging. For example, a clinician may desire to access one or more of the coronary artery ostia 30 via the aorta 20 (e.g., percutaneous coronary intervention (PCI) procedure). With reference to the simplified representation of FIG. 5B, where the neo-skirt 90 (represented by shading) blocks or partially impedes an intended path of a surgical device 92 from an interior of the previously-implanted bioprosthetic valve 40 (e.g., through a cell opening of the stent frame 52), access to one or more of the coronary artery ostia 30 is undesirably limited.

The coronary sequestration and access concerns associated with valve-in-valve arrangements are not limited to any particular type or design of bioprosthetic aortic valve or replacement transcatheter valve, and can arise with prosthetic valve constructions that differ from the general representations of FIGS. 4A-5B. Moreover, native patient anatomy and/or implant location of the initial bioprosthetic heart valve may also play a primary role in whether or not coronary sequestration and/or impediments to coronary access occur following deployment of a replacement transcatheter valve.

Against the above background, some embodiments of the present disclosure relate to systems (e.g., computing systems) and methods for evaluating a patient for risks associated with a potential valve-in-valve procedure. The systems and methods can be useful with different types or categories of patients. In some examples, embodiments of the present disclosure are useful with a first category of patients, such as those that have a previously-implanted prosthetic heart valve and having indications for receiving a candidate replacement transcatheter aortic valve for deployment within the previously-implanted bioprosthetic aortic valve. In other examples, embodiments of the present disclosure are useful with a second category of patients, such as those that are first time candidates for a bioprosthetic aortic valve (i.e., a bioprosthetic aortic valve has not yet been implanted to the patient). With this second category, prior to the patient receiving a first or initial bioprosthetic aortic valve, it can be useful to assess valve-in-valve risks presented by the first or initial bioprosthetic aortic valve under consideration. Under either scenario or patient category, then, evaluations of the present disclosure consider risks associated with potential deployment of a second or replacement transcatheter aortic valve within a first bioprosthetic aortic valve. For the first category of patients (i.e., those that have already received a bioprosthetic aortic valve), the "first bioprosthetic valve" is in reference to the previously-implanted bioprosthetic aortic valve. For the second category of patients (i.e., those that are first time candidates for receiving a bioprosthetic aortic valve), the "first bioprosthetic valve" is in reference to the bioprosthetic aortic valve under consideration.

In general terms, some methods of the present disclosure entail obtaining measurements of various anatomical features of the first bioprosthetic aortic valve relative to the patient's native anatomy. The obtained measurements are compared with benchmarks or determined measurements of the replacement transcatheter aortic valve deployed within a bioprosthetic aortic valve that is otherwise substantially identical to the first bioprosthetic aortic valve (e.g., the bioprosthetic aortic valve of the benchmark measurements is the same style/type/size as the first bioprosthetic aortic valve). From this review, an evaluation is made as to whether or not the replacement transcatheter aortic valve is appropriate for the patient, for example if the evaluation reveals a possible coronary sinus sequestration or coronary artery access obstruction risk or concern. With the systems and methods of the present disclosure, highly viable assessments or predictions of risks associated with a proposed valve-in-valve procedure, for example a transcatheter aortic valve-in-transcatheter aortic valve (or "TAV-in-TAV"), for a particular patient are provided.

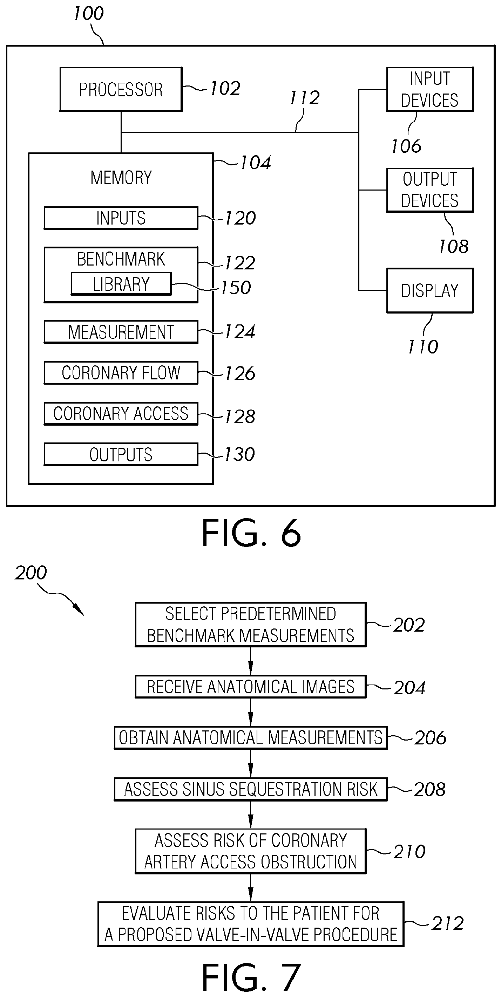

FIG. 6 is a block diagram illustrating a computing system 100 for evaluating a patient having indications for receiving a replacement transcatheter aortic valve within a first bioprosthetic aortic valve (e.g., a transcatheter aortic valve) according to one embodiment. The system 100 includes a processor 102, a memory 104, input devices 106, output devices 108, and a display 110. The processor 102, memory 104, input devices 106, output devices 108, and display 110 are communicatively coupled to each through a communication link 112.

The input devices 106 can include one or more of a keyboard, mouse, data ports, stylus and/or other suitable devices for inputting information into the system 100. The output devices 108 can include one or more of speakers, data ports, and/or outer suitable devices for outputting information from the system 100. The display 110 can be any type of display device that displays information to a user of the system 100.

The processor 102 includes a central processing unit (CPU) or other suitable processor. In an example, the memory 104 stores machine readable instructions executed by the processor 102 for operating the system 100. The memory 104 includes any suitable combination of volatile and/or non-volatile memory, such as combinations of random-access memory (RAM), read-only memory (ROM), flash memory, and/or other suitable memory. These are examples of non-transitory computer readable media (e.g., non-transitory computer-readable storage media storing computer-executable instructions that when executed by at least one processor cause the at least one processor to perform a method). The memory 104 is non-transitory in the sense that it does not encompass a transitory signal but instead is made up of at least one memory component to store machine executable instructions for performing techniques or methodologies described herein.

The memory 104 stores inputs 120, a benchmark module 122, a measurement module 124, a coronary flow evaluation module 126, a coronary access evaluation module 128, and outputs 130. Processor 102 executes instructions of modules 122, 124, 126, 128 to perform techniques described herein based on the inputs 120 to generate the outputs 130. In some embodiments, the inputs 120 include obtained images of a previously-implanted bioprosthetic aortic valve and surrounding anatomy of a patient. The benchmark module 122 selects, or facilitates user selection of, benchmark data or measurements corresponding with the replacement transcatheter aortic valve deployed within a bioprosthetic valve substantially identical to the first bioprosthetic aortic valve. The measurement module 124 obtains anatomical measurements of the first bioprosthetic aortic valve relative to native anatomy as described below. The coronary flow evaluation module 126 compares the obtained anatomical measurements with the selected benchmark data to assess sinus sequestration risks for the patient. The coronary access evaluation module 128 compares the obtained anatomical measurements with the selected benchmark data to assess coronary access risks for the patient. Results from one or more of the modules 122-128 can be provided to a user as the outputs 130.

In some examples, the various subcomponents or elements of the system 100 may be embodied in a plurality of different systems, whereas modules may be grouped or distributed across the plurality of different systems. To achieve its desired functionality, the system 100 may include various hardware components. Among these hardware components may be a number of processing devices, a number of data storage devices, a number of peripheral device adaptors, and a number of network adaptors. These hardware components may be interconnected through the use of busses and/or network connections. The processing devices may include a hardware architecture to retrieve executable code from the data storage devices and execute the executable code. The executable code may, when executed by the processing devices, cause the processing devices to execute some of the functionality disclosed herein.

FIG. 7 is a flow diagram illustrating a method 200 according to certain embodiments. In some embodiments, computing system 100 (FIG. 6) is configured to perform one or more or all steps of the method 200. It should be noted that in certain embodiments, method 200 is a computer-implemented method or process. Further, certain blocks may be performed automatically, manually by user of a computing device, or partially manually and partially automatically such as based on input from a user of a computing device. Further, certain blocks may be optional, and parts of the described method may be performed as separate methods. At 202, the method 200 includes selecting benchmark data or measurements are selected from a plurality of available, predetermined benchmark data or measurements based upon the replacement transcatheter aortic valve and the first bioprosthetic aortic valve. The selection at 202 may be performed by the benchmark module 122. At 204, the method includes receiving anatomical images for a patient. The images include or relate to an actual or potential location of the first bioprosthetic aortic valve and surrounding anatomy of the patient. At 206, the method includes obtaining anatomical measurements of the first aortic valve relative to native anatomy of the patient based on the obtained images. The measurements at 206 may be obtained by, or the generation of measurements may be facilitated by, the measurement module 124. At 208, the obtained anatomical measurements are compared with the selected benchmark measurements to assess sinus sequestration risks for the patient were the replacement transcatheter aortic valve to be installed or implanted within the first bioprosthetic aortic valve. The assessment at 208 can be performed by, or facilitated by, the coronary flow evaluation module 126. Optionally, at 210, the obtained anatomical measurements are compared with the selected benchmark measurements to assess coronary access risks for the patient were the replacement transcatheter aortic valve to be installed or implanted within the first bioprosthetic aortic valve. The assessment at 210 can be performed by, or facilitated by, the coronary access evaluation module 128. Based upon the assessment(s) at 208 and/or 210, an evaluation of risks to the patient for a valve-in-valve procedure is made at 212. The evaluation at 212 may be performed by one or both of the coronary flow evaluation module 126 and the coronary access evaluation module 128. Where the patient in question has previously received a bioprosthetic aortic valve and is under consideration for receiving a candidate replacement transcatheter aortic valve, the step 212 of evaluating can include determining whether the candidate replacement transcatheter aortic valve is appropriate for the patient. Where the patient in question has not previously received a bioprosthetic aortic valve and is under consideration for receiving a candidate initial bioprosthetic aortic valve, the step 212 of evaluating can include determining whether the candidate initial bioprosthetic aortic valve is appropriate for the patient.

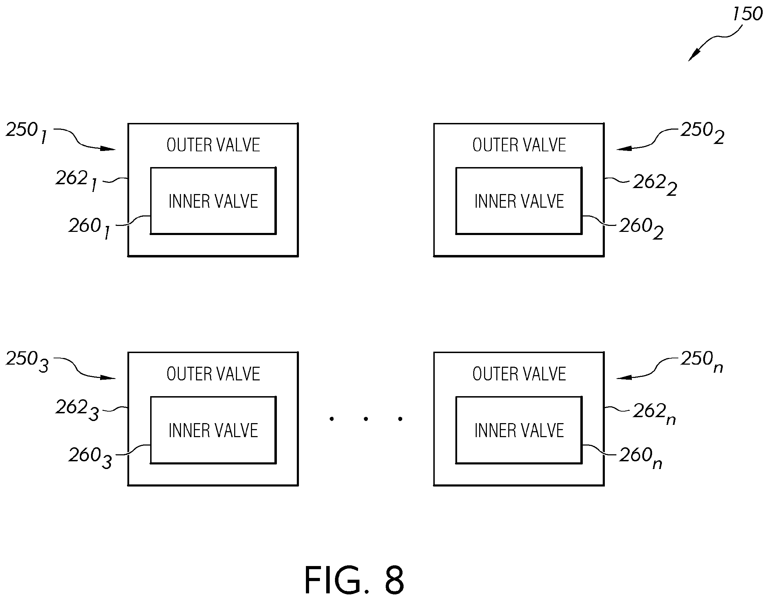

In some embodiments, the benchmark module 122 can have access to or maintain a library 150 of determined measurement data (e.g., obtained by bench testing) for at least one valve-in-valve ("VIV") combination of a known transcatheter aortic valve deployed within a known bioprosthetic aortic valve. In some embodiments, the library 150 includes or provides determined measurement data for a plurality of different VIV combinations. For example, FIG. 8 illustrates, in block form, a determined measurement data for a plurality of VIV combinations 250₁ ... 250*ₙ* that can be provided by the library 150. For ease of explanation, the two valves of each VIV combination 250₁ ... 250*ₙ* can be designated as an inner valve 260 deployed within an outer valve 262. The inner valve 260 of each of the VIV combinations 250₁ ... 250*ₙ* is a known transcatheter aortic valve (identifiable by at least type or trade designation and size). A variety of different transcatheter aortic valves are currently available, each with certain design features. Some examples include transcatheter aortic valves available under the trade designation CoreValve^{™} from Medtronic, Inc., Evolut^{™} from Medtronic, Inc., Sapien^{™} from Edwards Lifesciences, Inc., Portico^{™} from Abbott, Acurate^{™} Neo from Boston Scientific, etc. These and other transcatheter aortic valves are available in different, designated sizes. Thus, the inner valve 260₁ of the first VIV combination 250₁ can be an Evolut^{™} PRO 26 millimeter transcatheter aortic valve; the inner valve 260₂ of the second VIV combination 250₂ can be an Evolut PRO 29 millimeter transcatheter aortic valve; the inner valve 260₃ can be a transcatheter aortic valve of a designated size available from a manufacturer other than Medtronic, Inc.; etc. The outer valve 262 of each of the VIV combinations 250₁ ... 250*ₙ* is a known bioprosthetic aortic valve that may or may not be a known transcatheter aortic valve (e.g., the outer valve 262 of one or more of the VIV combinations 250₁ ... 250*ₙ* may be instead be a surgical prosthetic aortic valve). At least some of the VIV combinations 250₁ ... 250*ₙ* provide determined measurement information for a known transcatheter aortic valve deployed within a known transcatheter aortic valve (and are thus representative of a transcatheter aortic valve-in-transcatheter aortic valve (or "TAV-in-TAV") valve replacement arrangement). The VIV combinations 250₁ ... 250*ₙ* can include determined measurement information for a known transcatheter aortic valve deployed with the same known transcatheter aortic valve. For example, with the VIV combination 250₁, the inner valve 260₁ and the outer valve 262₁ are the same make, model and size. The VIV combinations 250₁ ... 250*ₙ* can include determined measurement information for a known transcatheter aortic valve of a first size deployed within a known transcatheter aortic valve similar to, but differently sized from, a known transcatheter aortic valve. For example, with the VIV combination 250₂, the inner valve 260₂ and the outer valve 262₂ have the same make and model, but differ in size. The VIV combinations 250₁ ... 250*ₙ* can include determined measurement information for a known transcatheter aortic valve from a first manufacturer deployed within a known transcatheter aortic valve from a second manufacturer. For example, with the VIV combination 250₃, the inner valve 260₃ is a known transcatheter aortic valve produced by a first manufacturer and the outer valve 262₂ is a known transcatheter aortic valve produced by a different manufacturer. Determined measurement data for a wide variety of VIV combinations can be provided.

Returning to FIGS. 6 and 7, the determined measurement data can include various dimensional attributes associated with each VIV combination, for example measurements representing a height of pinned leaflets (or neo-skirt) relative to one or more points of interest, such as the inflow end of the known bioprosthetic aortic valve, a marker on the known bioprosthetic aortic valve, etc. In some embodiments, the measurement data can be provided relative to a plane at which an inflow end of the known bioprosthetic aortic valve is expected to be located upon final implant (e.g., plane of the native aortic valve annulus). Other determined measurement data can include a diameter of the combination known transcatheter aortic valve deployed within a known bioprosthetic aortic valve at one or more locations, for example at an extent or level of the pinned leaflets or neo-skirt.

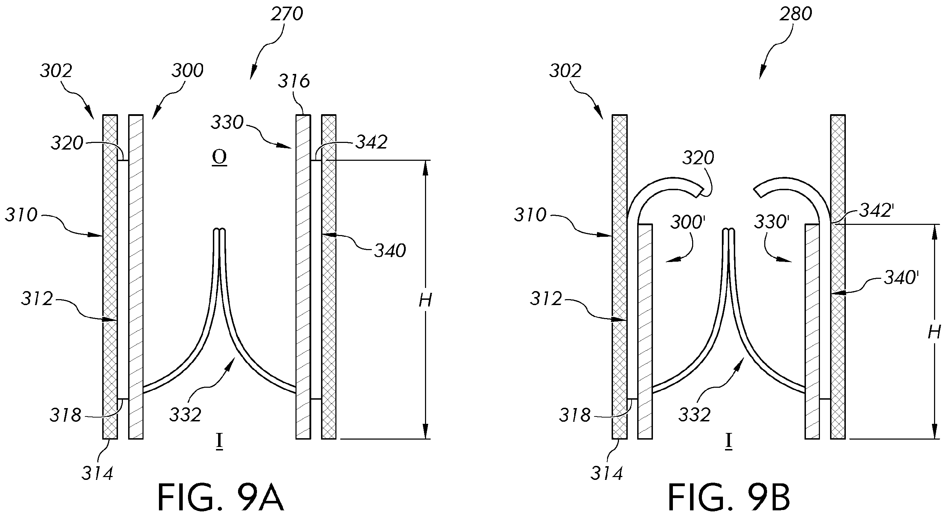

As a point of reference, FIG. 9A is a simplified representation of a benchmarking VIV combination 270 of a known transcatheter aortic valve 300 deployed within a known bioprosthetic aortic valve 302 and from which measurement data useful with the systems and methods of the present disclosure can be determined. The known bioprosthetic aortic valve 302 includes a stent frame 310 and leaflets 312 (a thickness of which is exaggerated for ease of understanding) that have been pinned by a stent frame 330 of the known transcatheter aortic valve 300 upon final deployment, thus creating a neo-skirt 340. Leaflets 332 of the known transcatheter aortic valve 300 are also reflected in FIG. 9A; an arrangement of the leaflets 332 establishes an inflow side I opposite an outflow side O. The leaflets 312 of the known bioprosthetic aortic valve 302 are similarly arranged relative to the stent frame 310 such that the known bioprosthetic aortic valve 302 has the same inflow and outflow sides I, O. Thus, and commensurate with the descriptions above, the stent frame 310 of the known bioprosthetic aortic valve 302 has an inflow end 314 opposite an outflow end 316, with the leaflets 312 extending from a base 318, that is otherwise secured to the stent frame 310, to a free margin 320. With the arrangement of FIG. 9A, an entire extent or length of the leaflets 312 are pinned between the stent frames 310, 330 including the free margin 320. Thus, a pinned edge 342 of the neo-skirt 340 is established at the free margin 320. With these conventions in mind, a height H of the neo-skirt 340 can be measured as the length or distance from the inflow end 314 to the pinned edge 342. Alternatively or in addition, the height H of the neo-skirt 340 can be measured as the length or distance from a marker or other known location along the stent frame 310 near the inflow side I (e.g., at or near the base 318 of the leaflets 312) to the pinned edge 342.

As a point of further reference, FIG. 9B illustrates, in simplified form, another benchmarking VIV combination 280 of a different, known transcatheter aortic valve 300' deployed within the known bioprosthetic aortic valve 302 in a manner creating a neo-skirt 340'. A stent frame 330' of the known transcatheter aortic valve 300' is substantively shorter than the stent frame 310 of the known bioprosthetic aortic valve 302. In the arrangement of FIG. 9B, then, less than an entire length of the leaflets 312 are pinned between the stent frames 310, 330'. While the base 318 is between the stent frames 310, 330', the free margin 320 is not. An extent of the stent frame 330' generates a pinned edge 342' along the leaflets 312. With the partially pinned arrangement, a height H of the resulting neo-skirt 340' can be measured as the length or distance from the inflow end 314 to the pinned edge 342'. Alternatively or in addition, the height H of the neo-skirt 340' can be measured as the length or distance from a marker or other known location along the stent frame 310 near the inflow side I to the pinned edge 342'.

Returning to FIGS. 6 and 7, in some embodiments, the determined measurement data maintained by the library 150 and/or otherwise accessible by the benchmark library module 124 can account for various depths of implant. As a point of reference, in that the determined measurement data, including the neo-skirt height, will be used for evaluating an actual, previously-implanted bioprosthetic aortic valve relative to surrounding anatomy, the "depth of implant" is in reference to a location of an implanted bioprosthetic aortic valve relative to native anatomy, for example a distance between an inflow end of an implanted bioprosthetic aortic valve and a plane of the native aortic valve annulus. The depth of implant can, and often does, vary from patient to patient. In that the determined measurement data, including the neo-skirt height, will be used for evaluating an actual, implanted bioprosthetic aortic valve, the determined measurement data can provide for two or more potential depths of implant.

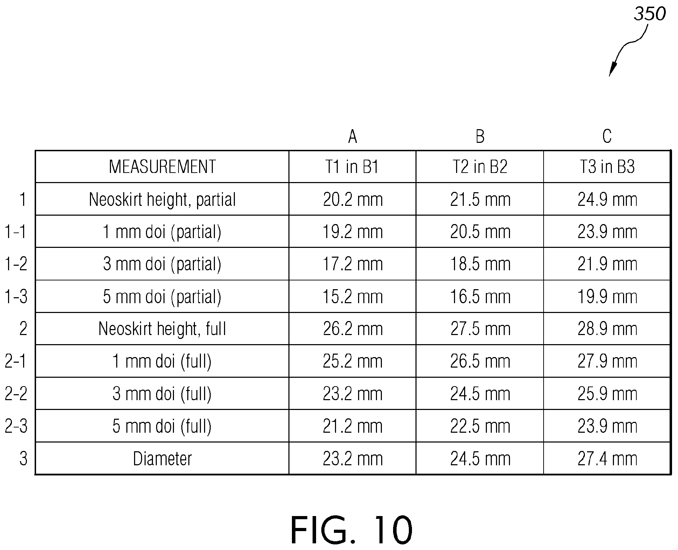

With the above in mind, the determined measurement data can assume various forms, and can include benchmark information for two or more combinations of a known transcatheter aortic valve deployed within a known bioprosthetic aortic valve (e.g., obtained by bench testing). One non-limiting example of determined measurement data or lookup table 350 is provided in FIG. 10. The determined measurement data includes benchmark information for a first known transcatheter aortic valve T1 deployed within a first known bioprosthetic aortic valve B1 (column A), a second known transcatheter aortic valve T2 deployed within a second known bioprosthetic aortic valve B2 (column B), and a third known transcatheter aortic valve T3 deployed within a third known bioprosthetic aortic valve B3 (column C). The benchmark testing utilized to generate the determined measurement data 350 can include arranging the known transcatheter aortic valve relative to the corresponding known bioprosthetic valve such that the leaflets of the known bioprosthetic valve are either partially pinned or fully pinned. With this in mind, benchmark measurements provide for the neo-skirt height with partially pinned leaflets (row 1), neo-skirt height with fully pinned leaflets (row 2), and diameter at the pinned edge of the neo-skirt (row 3). In addition, the determined measurement data 400 can include the neo-skirt height (for both partially and fully pinned conditions) relative to different depths of implant, for example a depth of implant of 1 millimeter (rows 1-1 and 2-1), a depth of implant of 3 millimeters (rows 1-2 and 2-2), and a depth of implant of 5 millimeters (rows 1-3 and 2-3). The determined measurement data of the present disclosure can assume a wide variety of other forms.

With additional reference to FIGS. 6 and 7, the step 202 can include selecting determined measurement data from the library 150 that corresponds with the first bioprosthetic aortic valve of the patient and the replacement transcatheter aortic valve under consideration. For example, where the first bioprosthetic aortic valve (e.g., the previously-implanted bioprosthetic aortic valve for a patient that has already received a bioprosthetic aortic valve, a bioprosthetic aortic valve under consideration for a first time candidate patient) is the known bioprosthetic aortic valve B2 and the replacement transcatheter aortic valve is the known transcatheter aortic valve T2, the measurement data provided by column B is selected.

The anatomy images of the patient provided at step 204 can be obtained in various manners. In some embodiments, data representative of patient-specific, three-dimensional (3D) images of a cardiac region at which the first bioprosthetic aortic valve has been, or potentially will be, implanted is provided to the processor 102, for example obtained by computer tomography (CT) or magnetic resonance imaging (MRI). Thus, the data can be one or more 3D CT images and/or one or more 3D MRI images of the cardiac region of the subject. Thus, in some embodiments, the inputs 120 can include a medical image device and/or a database of obtained medical images (e.g., single phase CT images or multiphase CT images imported to the system 100). Where the patient in question has previously received a bioprosthetic aortic valve, the previously-implanted bioprosthetic aortic valve will be present in the obtained images.

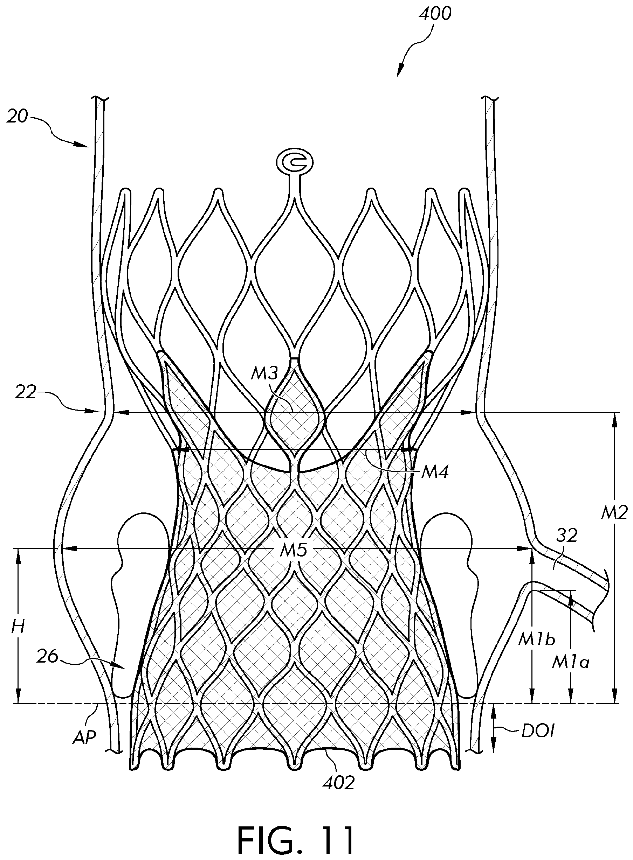

The step 206 of obtaining anatomical measurements of the first bioprosthetic aortic valve relative to native anatomy of the patient in the obtained images can include or incorporate various techniques or processes that generate information useful for subsequent evaluation. For example, anatomical measurements can include one or more of coronary height from the inflow, residual distance, diameters or other parameters indicative of area or volume between the previously-implanted bioprosthetic valve and native anatomy (e.g., aortic wall) at one or more locations, commissure alignment, etc. With reference to FIG. 11, that otherwise illustrates a previously-implanted bioprosthetic aortic valve 400 and surrounding anatomy in some non-limiting examples, the anatomical measurements can include a first measurement providing the height or distance of each of the coronary artery ostia 32 from the native annulus 26. The ostium height measurement can be one or both of an inferior ostium height M1a and a superior ostium height M1b. The anatomical measurements can further include a second measurement M2 providing the height or distance of the sinotubular junction ("STJ") 22 from the native annulus 26, a third measurement M3 providing the diameter of the STJ 22, a fourth measurement M4 providing the diameter of the previously-implanted valve 400 at the STJ 22, and a fifth measurement M5 providing a parameter indicative of size and/or shape of the aorta or aortic wall 20 (or other anatomy) at a distance from the native annulus 26 corresponding with the neo-skirt height H obtained from the benchmark measurement data. For example, a parameter of the fifth measurement M5 can be a diameter, residual area, residual volume, etc. Regarding the fifth measurement M5, a depth of implant DOI of the previously-implanted valve 400 (i.e., distance from an inflow end 402 of the previously-implanted valve 400 to the annular plane AP of the native annulus 26) can be measured or determined; the DOI is compared with the retrieved neo-skirt height benchmark measurements (that otherwise correspond with the replacement transcatheter aortic valve deployed within a bioprosthetic aortic valve that is substantially identical to the previously-implanted valve 400) to select a corresponding neo-skirt height H, optionally for both fully pinned and partially pinned arrangements if available. In other embodiments, methods of the present disclosure can default to a 3 millimeter depth of implant DOI. Regardless, the obtained neo-skirt height H is then used to determine a location (e.g., distance from the annular plane AP) at which the fifth measurement M5 is determined.

For patients with a previously-implanted bioprosthetic aortic valve, the measurements described above can be obtained relative to the actual position and orientation of the previously-implanted bioprosthetic aortic valve. For first time candidate patients (i.e., a patient who has not yet received a first bioprosthetic aortic valve and thus a first or previously-implanted bioprosthetic aortic valve is not present in the obtained anatomical images), the fifth measurement M5 can be obtained by measuring the parameter indicative of size and/or shape (e.g., diameter, area, volume, etc.) of the native aorta 20 at the height H or plane where the pinned leaflet is estimated to be. Once again, the estimation is made by using the neoskirt height H obtained from the benchmark measurement data relative to an expected or planned depth of implant DOI.

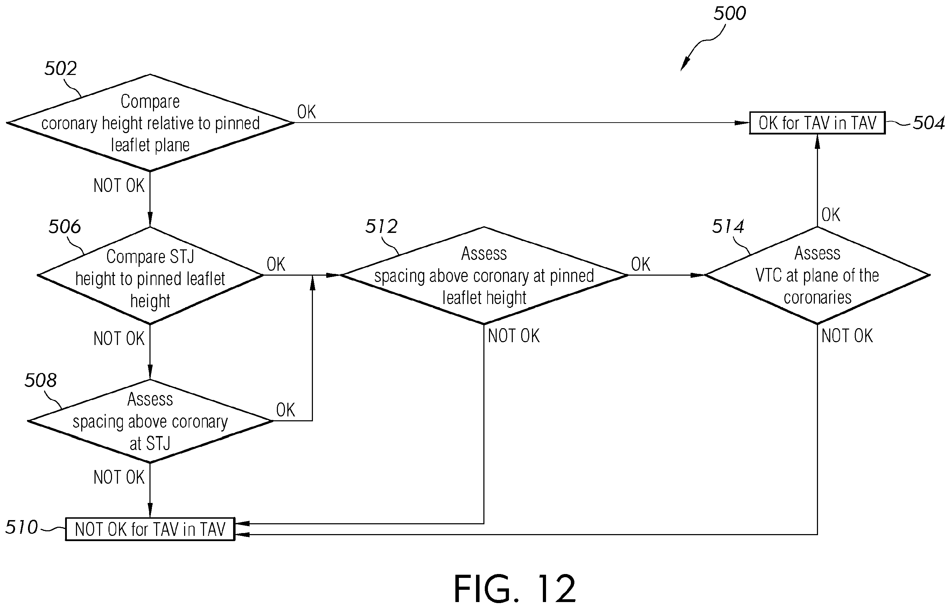

Returning to FIGS. 6 and 7, the step 208 of comparing the obtained anatomical measurements with the selected benchmark measurements to assess sinus sequestration risks for the patient were the candidate replacement transcatheter aortic valve to be installed or implanted within the first bioprosthetic aortic valve can include or incorporate various techniques or processes. One non-limiting example of a coronary flow assessment method 500 for a patient with a previously-implanted bioprosthetic aortic valve is provided in FIG. 12. With additional reference to FIG. 11, at step 502, the coronary artery ostium height (inferior height measurement M1a, superior height measurement M1b, or both) is compared with the benchmark neo-skirt (or pinned leaflet) height H for both of the coronary arteries. Under circumstances where all coronary artery ostia heights exceed the benchmark neo-skirt height H by a predetermined value ("OK" at step 502), for example 2 millimeters, it can be determined that there is a low risk for sinus sequestration and the patient can be preliminarily approved for receiving the candidate replacement transcatheter aortic valve at step 504.

If the coronary artery height does not exceed the neo-skirt height H ("NOT OK" at step 502), the STJ height (measurement M2) is compared with the benchmark neo-skirt (or pinned leaflet) height H at step 506. Under circumstances where this comparison reveals that the STJ height is less than the neo-skirt height H ("NOT OK" at step 506), a parameter indicative of spacing between the previously-implanted valve and the aorta at the sinotubular junction STJ is assessed at step 508. The assessed parameter can be valve to aorta distance ("VTA"), residual area, residual volume, etc. For example, the sinotubular junction STJ diameter (measurement M3) can be compared with the diameter of the previously-implanted valve at a level of the sinotubular junction STJ (measurement M4). Under circumstances where the STJ diameter is determined to not exceed the diameter of the previously-implanted valve at the level of the STJ by a predetermined value ("NOT OK" at step 508), for example 3 millimeters, it can be determined that there is a heightened risk for sinus sequestration and the patient can be preliminarily disapproved for receiving the candidate replacement transcatheter aortic valve at step 510.

If the STJ height (M2) is greater than the benchmark neo-skirt height H ("OK" at step 506) or the STJ diameter (M3) is determined to exceed the diameter of the previously-implanted valve at the level of the STJ 22 ("OK" at step 508), a parameter indicative of spacing between the previously-implanted valve and the aorta 20 (or other native anatomy) at a level of the neo-skirt (or pinned leaflet) height H is assessed at step 512. The assessed parameter can be valve to aorta distance ("VTA"), residual area, residual volume, etc. For example, the diameter of the aortic wall 20 (or other anatomy) at a distance from the native annulus 26 corresponding with the neo-skirt height H (measurement M5) can be compared with the benchmark diameter. Under circumstances where the aortic wall 20 (or other anatomy) diameter at the neo-skirt height H does not exceed the benchmark diameter by a predetermined value ("NOT OK" at step 512), for example 3 millimeters, it can be determined that there is a heightened risk for sinus sequestration and the patient can be preliminarily disapproved for receiving the candidate replacement transcatheter aortic valve at step 510.

Under circumstances where the aortic wall (or other anatomy) diameter at the neo-skirt height H exceeds the benchmark diameter by a predetermined value ("OK" at step 512), for example 3 millimeters, a residual or open area or distance between the previously-implanted valve and each of the coronary artery ostia at a plane of the coronary ostia ("VTC") is assessed at step 514. For example, a distance between the previously-implanted valve and the ostium of each of the coronary arteries can be determined and compared with a benchmark distance. Under circumstances where the VTC relative to each of the coronary artery ostia greater than the benchmark distance, for example 3 millimeters, it can be determined that there is a low risk for sinus sequestration and the patient can be preliminarily approved for receiving the candidate replacement transcatheter aortic valve at step 504. Conversely, under circumstances where the VTC relative to each of the coronary artery ostia does not exceed the benchmark distance, it can be determined that there is a heightened risk for sinus sequestration and the patient can be preliminarily disapproved for receiving the candidate replacement transcatheter aortic valve at step 510.

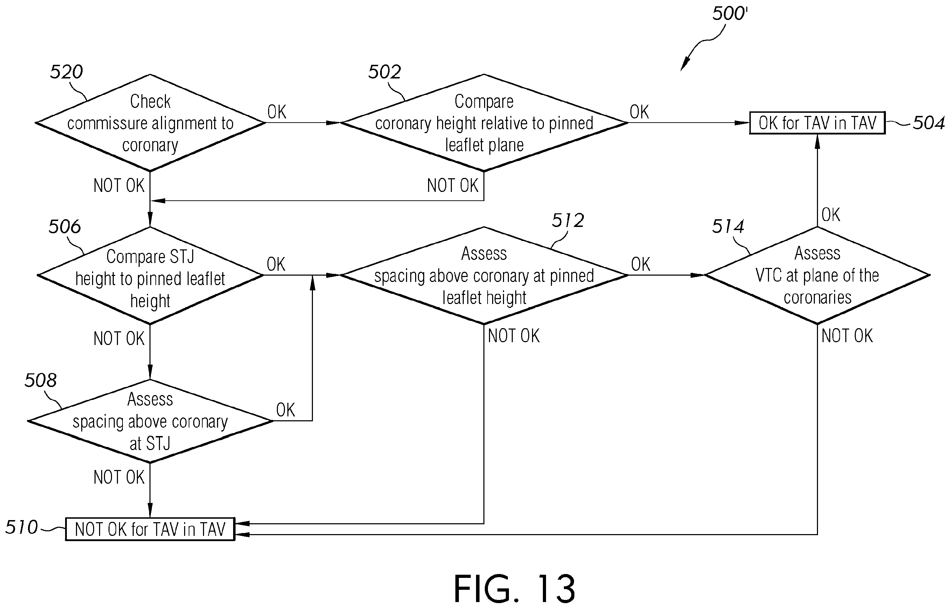

The methods for assessing or evaluating risk of sinus sequestration for a patient with a previously-implanted bioprosthetic aortic valve of the present disclosure can include one or more steps in addition to, or as an alternative to, one or more of the steps of the method 500. For example, FIG. 13 illustrates an alternative method 500' for a patient with a previously-implanted bioprosthetic aortic valve that further includes the optional step 520 of reviewing an alignment of commissures of the previously-implanted valve relative to the coronary artery ostia. Where the commissures are found to be sufficiently offset from the ostia ("OK" at step 520), for example by at least 20 degrees, then the coronary artery ostium height (measurement M1a, M1b, or both) is compared with the benchmark neo-skirt (or pinned leaflet) height H for both of the coronary arteries at step 502 as described above. Where one or more of commissures are determined to be closely aligned with one or more of the coronary artery ostia ("NOT OK" at step 520), then the STJ height (measurement M2) is compared with the benchmark neo-skirt (or pinned leaflet) height H at step 506 as described above. A remainder of the method 500' can be similar to the method 500.

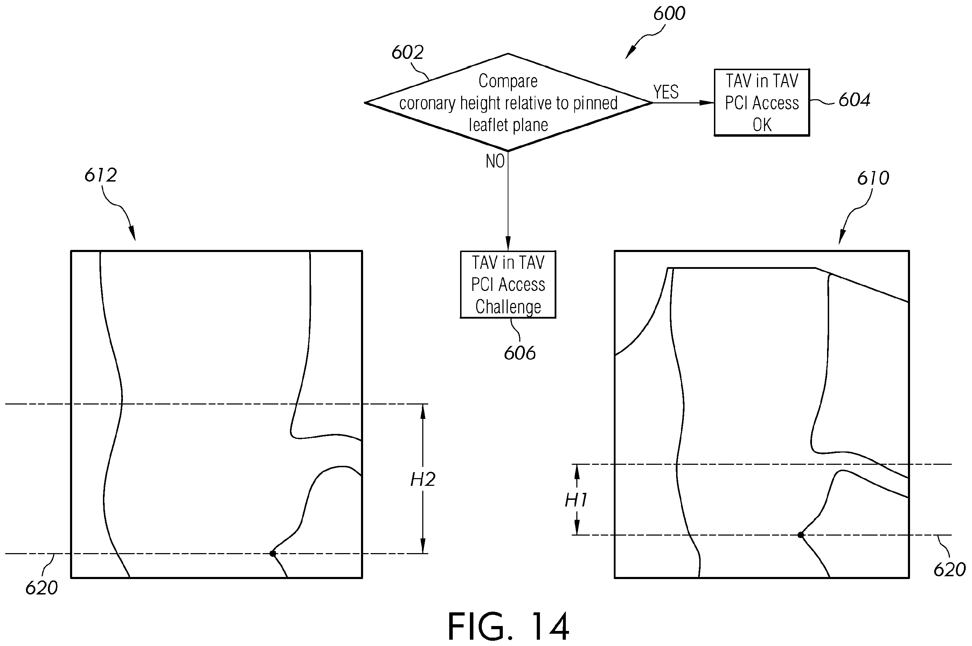

Returning to FIGS. 6 and 7, the step 210 of comparing the obtained anatomical measurements with the selected benchmark measurements to assess coronary access risks can include or incorporate various techniques or processes. One non-limiting example of a coronary access assessment method 600 for a patient with a previously-implanted bioprosthetic heart valve is provided in FIG. 14. With additional reference to FIG. 11, at step 602, the coronary artery ostium height (inferior height measurement M1a, superior height measurement M1b, or both) is compared with the benchmark neo-skirt (or pinned leaflet) height H for both of the coronary arteries. Under circumstances where all coronary artery ostia heights are greater than the benchmark neo-skirt height H ("Yes" at step 602), it can be determined that there is a low risk for coronary access concerns and the candidate replacement transcatheter aortic valve can be designated as presenting minimal obstacles to percutaneous coronary intervention (PCI) procedures at step 604. As a point of reference, image 610 is an example comparison in which a benchmark neo-skirt height or plane H1 is less than or "below" the superior aspect of a coronary ostium 620. Under circumstances where at least one coronary artery ostium height is less than the benchmark neo-skirt height H ("No" at step 602), it can be determined that there is an increased risk for coronary access concerns and the candidate replacement transcatheter aortic valve can be designated as presenting minimal obstacles to percutaneous coronary intervention (PCI) procedures at step 606. As a point of reference, image 612 is an example comparison in which a benchmark neo-skirt height or plane H2 is greater than or "above" the superior aspect of the coronary ostium 620. Other, optional coronary assessment methods of the present disclosure can include modeling coronary flow based on anatomy and bench measurements.

Commensurate with the descriptions above, the methods of, and akin to, those of FIGS. 12-14 can be appropriate for patients with a previously-implanted bioprosthetic aortic valve, for example to assess or evaluate risks of a potential valve-in-valve procedure. In other embodiments, the systems and methods of the present disclosure can be useful for first time bioprosthetic aortic valve candidate patients (i.e., patients that are under consideration for, but have not yet received, a bioprosthetic aortic valve). For this category of patients, methods of the present disclosure can be akin to the methods 500, 500' of FIGS. 12 and 13, with the comparisons or assessments at steps 502, 506, 508, and 512 being made relative to anatomical measurements and bench-measured dimensions. The step 514 of assessing VTC need not be performed. Regardless, where the first time patient, valve-in-valve assessment for a candidate first bioprosthetic aortic valve reveals an elevated risk of sinus sequestration, the clinician may select a different candidate first bioprosthetic aortic valve for the first time patient (e.g., the clinician may select a different (likely shorter) first bioprosthetic aortic valve if the valve-in-valve risk assumed at baseline is too high with a supraannular valve).

It should be understood that various aspects disclosed herein may be combined in different combinations than the combination specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purpose of clarity, it should be understood that the techniques of this disclosure may be performed by a combination of units or modules associated with, for example, a medical device.

In one or more examples, the described techniques may be implemented in hardware, software, firmware, or a combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The systems and methods of the present disclosure provide a marked improvement over previous designs. By utilizing methodologies that compare measurements, for example TAV in TAV measurements, from benchmark testing to a patient's anatomy, reliable evaluations or screening of patients for replacement valve procedures can be made.

Although the present disclosure has been described with reference to preferred embodiments, persons skilled in the art will recognize that changes can be made in form and detail without departing from the scope of the present disclosure.

## Claims

1. A computer-implemented method for evaluating a proposed valve-in-valve procedure for a patient in which a replacement transcatheter aortic valve (70) will be deployed within a first bioprosthetic aortic valve (40), the method comprising:
selecting (202) predetermined benchmark measurements of a valve-in-valve combination comprising a known transcatheter aortic valve deployed within a known bioprosthetic aortic valve;
receiving (204) images of an anatomy of the patient;
obtaining (206) anatomical measurements of the first bioprosthetic valve from the received images;
reviewing the predetermined benchmark measurements and the anatomical measurements; and
evaluating (212) risks of a valve-in-valve procedure for the patient based, at least in part, upon the review.

2. The method of claim 1, wherein the first bioprosthetic aortic valve is a bioprosthetic aortic valve previously implanted within the patient, and further wherein the step of evaluating includes determining whether the replacement transcatheter aortic valve is appropriate for the patient; or wherein the patient has not yet received a bioprosthetic aortic valve, and further wherein the first bioprosthetic aortic valve is a bioprosthetic aortic valve under consideration for implanting into the patient.

3. The method of claim 1, wherein the first bioprosthetic aortic valve is a transcatheter aortic valve; or wherein the known transcatheter aortic valve is substantially identical to the candidate transcatheter aortic valve, and the known bioprosthetic aortic valve is substantially identical to the first bioprosthetic aortic valve.

4. The method of claim 1, wherein the step of evaluating includes assessing (208) a risk of sinus sequestration; or wherein the step of evaluating includes assessing (210) a risk of coronary artery access obstruction.

5. The method of claim 1, wherein the valve-in-valve combination defines a neo-skirt, and further wherein the predetermined benchmark measurements include a height of the neo-skirt and a diameter of the valve-in-valve combination.

6. The method of claim 5, wherein the neo-skirt is generated by leaflets of the known bioprosthetic aortic valve pinned between a stent frame of the bioprosthetic aortic valve and a stent frame of the known transcatheter aortic valve.

7. The method of claim 6, wherein the leaflets of the known bioprosthetic aortic valve are one of fully pinned and partially pinned between the stent frames.

8. The method of claim 6, wherein the height of the neo-skirt is defined by a distance from an inflow end of the stent frame of the known bioprosthetic aortic valve and a pinned edge of the leaflets; or wherein the diameter is obtained at a plane of a pinned edge of the neo-skirt.

9. The method of claim 1, wherein first bioprosthetic aortic valve is a previously-implanted bioprosthetic aortic valve, and the anatomical measurements include a distance from each native coronary artery ostium to an inflow end of the previously-implanted bioprosthetic aortic valve.

10. The method of claim 9, wherein the anatomical measurements further include a distance from the inflow end to a native sinotubular junction.

11. The method of claim 9, wherein the anatomical measurements further include a parameter indicative of a spacing between the previously-implanted bioprosthetic aortic valve and a native aortic wall; and optionally wherein the parameter is selected from the group consisting of a distance, a residual area, and a residual volume.

12. The method of claim 1, wherein the step of reviewing includes comparing a neo-skirt height value of the predetermined benchmark measurements with a coronary artery ostium height value of the anatomical measurements; or wherein the step of reviewing includes comparing a neo-skirt height value of the predetermined benchmark measurements with a sinotubular junction height of the anatomical measurements.

13. The method of claim 1, wherein the step of reviewing includes assessing a parameter indicative of a spacing between the first bioprosthetic aortic valve and native anatomy at a level of a native sinotubular junction; and optionally wherein the parameter is selected from the group consisting of a distance, a residual area, and a residual volume.

14. The method of claim 1, wherein the step of reviewing includes assessing a parameter indicative of a spacing between the first bioprosthetic aortic valve and native anatomy at a level corresponding with a neo-skirt height value of the predetermined benchmark measurements; and optionally wherein the parameter is selected from the group consisting of a distance, a residual area, and a residual volume.

15. The method of claim 1, wherein the predetermined benchmark measurements of a valve-in-valve combination are obtained via bench testing; or wherein the predetermined benchmark measurements are obtained from a library, and further wherein the library maintains predetermined benchmark measurements for a plurality of different valve-in-valve combinations.

## Patentansprüche

1. Computerimplementiertes Verfahren zum Bewerten einer vorgeschlagenen Klappe-in-Klappe-Prozedur bei einem Patienten, bei dem eine Ersatz-Transkatheter-Aortenklappe (70) in eine erste bioprothetische Aortenklappe (40) eingesetzt wird, wobei das Verfahren umfasst:
Auswählen (202) vorbestimmter Referenzmessungen einer Klappe-in-Klappe-Kombination, die eine bekannte Transkatheter-Aortenklappe umfasst, die in eine bekannte bioprothetische Aortenklappe eingesetzt ist;
Empfangen (204) von Bildern einer Anatomie des Patienten;
Erhalten (206) anatomischer Messungen der ersten bioprothetischen Klappe aus den empfangenen Bildern;
Überprüfen der vorbestimmten Referenzmessungen und der anatomischen Messungen; und
Bewerten (212) von Risiken einer Klappe-in-Klappe-Prozedur für den Patienten, zumindest teilweise auf Grundlage der Überprüfung.

2. Verfahren nach Anspruch 1, wobei die erste bioprothetische Aortenklappe eine zuvor in den Patienten implantierte bioprothetische Aortenklappe ist, und ferner wobei der Schritt des Bewertens das Bestimmen einschließt, ob die Ersatz-Transkatheter-Aortenklappe für den Patienten geeignet ist; oder wobei der Patient noch keine bioprothetische Aortenklappe erhalten hat, und ferner wobei die erste bioprothetische Aortenklappe eine bioprothetische Aortenklappe ist, deren Implantation in den Patienten in Betracht gezogen wird.

3. Verfahren nach Anspruch 1, wobei die erste bioprothetische Aortenklappe eine Transkatheter-Aortenklappe ist; oder wobei die bekannte Transkatheter-Aortenklappe im Wesentlichen mit der Kandidaten-Transkatheter-Aortenklappe identisch ist und die bekannte bioprothetische Aortenklappe im Wesentlichen mit der ersten bioprothetischen Aortenklappe identisch ist.

4. Verfahren nach Anspruch 1, wobei der Schritt des Bewertens das Auswerten (208) eines Risikos einer Sinus-Sequestrierung einschließt; oder wobei der Schritt des Bewertens das Auswerten (210) eines Risikos einer Obstruktion des Koronararterienzugangs einschließt.

5. Verfahren nach Anspruch 1, wobei die Klappe-in-Klappe-Kombination eine Neo-Schürze definiert und ferner wobei die vorbestimmten Referenzmessungen eine Höhe der Neo-Schürze und einen Durchmesser der Klappe-in-Klappe-Kombination einschließen.

6. Verfahren nach Anspruch 5, wobei die Neo-Schürze durch Segel der bekannten bioprothetischen Aortenklappe gebildet wird, die zwischen einem Stentrahmen der bioprothetischen Aortenklappe und einem Stentrahmen der bekannten Transkatheter-Aortenklappe angeheftet sind.

7. Verfahren nach Anspruch 6, wobei die Segel der bekannten bioprothetischen Aortenklappe zwischen den Stentrahmen entweder vollständig angeheftet oder teilweise angeheftet sind.

8. Verfahren nach Anspruch 6, wobei die Höhe der Neo-Schürze durch einen Abstand zwischen einem Einlassende des Stentrahmens der bekannten bioprothetischen Aortenklappe und einer angehefteten Kante der Segel definiert ist; oder wobei der Durchmesser in einer Ebene einer angehefteten Kante der Neo-Schürze erhalten wird.

9. Verfahren nach Anspruch 1, wobei die erste bioprothetische Aortenklappe eine zuvor implantierte bioprothetische Aortenklappe ist und die anatomischen Messungen einen Abstand von jedem Ostium einer nativen Koronararterie zu einem Einlassende der zuvor implantierten bioprothetischen Aortenklappe einschließen.

10. Verfahren nach Anspruch 9, wobei die anatomischen Messungen ferner einen Abstand von dem Einlassende zu einem nativen sinotubulären Übergang einschließen.

11. Verfahren nach Anspruch 9, wobei die anatomischen Messungen ferner einen Parameter einschließen, der einen Abstand zwischen der zuvor implantierten bioprothetischen Aortenklappe und einer nativen Aortenwand angibt; und wobei der Parameter optional ausgewählt ist aus der Gruppe bestehend aus einem Abstand, einer Restfläche und einem Restvolumen.

12. Verfahren nach Anspruch 1, wobei der Schritt des Überprüfens das Vergleichen eines Wertes der Höhe der Neo-Schürze der vorbestimmten Referenzmessungen mit einem Wert der Höhe des Ostiums der Koronararterie der anatomischen Messungen einschließt; oder wobei der Schritt des Überprüfens das Vergleichen eines Wertes der Höhe der Neo-Schürze der vorbestimmten Referenzmessungen mit einer Höhe des sinotubulären Übergangs der anatomischen Messungen einschließt.

13. Verfahren nach Anspruch 1, wobei der Schritt des Überprüfens das Auswerten eines Parameters einschließt, der einen Abstand zwischen der ersten bioprothetischen Aortenklappe und der nativen Anatomie auf Höhe eines nativen sinotubulären Übergangs angibt; und wobei der Parameter optional ausgewählt ist aus der Gruppe bestehend aus einem Abstand, einer Restfläche und einem Restvolumen.

14. Verfahren nach Anspruch 1, wobei der Schritt des Überprüfens das Auswerten eines Parameters einschließt, der einen Abstand zwischen der ersten bioprothetischen Aortenklappe und der nativen Anatomie auf einer Höhe angibt, die einem Höhenwert der Neo-Schürze der vorbestimmten Referenzmessungen entspricht; und wobei der Parameter optional ausgewählt ist aus der Gruppe bestehend aus einem Abstand, einer Restfläche und einem Restvolumen.

15. Verfahren nach Anspruch 1, wobei die vorbestimmten Referenzmessungen einer Klappe-in-Klappe-Kombination über Benchtests erhalten werden; oder wobei die vorbestimmten Referenzmessungen aus einer Bibliothek erhalten werden und ferner wobei die Bibliothek vorbestimmte Referenzmessungen für eine Vielzahl verschiedener Klappe-in-Klappe-Kombinationen führt.

## Revendications

1. Procédé mis en œuvre par ordinateur
permettant d'évaluer une intervention de valve dans valve proposée pour un patient dans laquelle une valve aortique transcathéter de remplacement (70) sera déployée à l'intérieur d'une première valve aortique bioprothétique (40), le procédé comprenant :
la sélection (202) de mesures de référence prédéterminées d'une combinaison valve dans valve comprenant une valve aortique transcathéter connue déployée dans une valve aortique bioprothétique connue ;
la réception (204) d'images d'une anatomie du patient ;
l'obtention (206) de mesures anatomiques de la première valve bioprothétique à partir des images reçues ;
l'examen des mesures de référence prédéterminées et des mesures anatomiques ; et
l'évaluation (212) de risques d'une intervention valve-in-valve pour le patient sur la base, au moins en partie, de l'examen.

2. Procédé selon la revendication 1, dans lequel la première valve aortique bioprothétique est une valve aortique bioprothétique préalablement implantée chez le patient, et dans lequel en outre l'étape d'évaluation comprend le fait de déterminer si la valve aortique transcathéter de remplacement est appropriée pour le patient ; ou dans lequel le patient n'a pas encore reçu de valvule aortique bioprothétique, et en outre dans lequel la première valvule aortique bioprothétique est une valve aortique bioprothétique envisagée pour une implantation chez le patient.

3. Procédé selon la revendication 1, dans lequel la première valve aortique bioprothétique est une valve aortique transcathéter ; ou dans lequel la valve aortique transcathéter connue est sensiblement identique à la valve aortique transcathéter candidate, et la valve aortique bioprothétique connue est sensiblement identique à la première valve aortique bioprothétique.

4. Procédé selon la revendication 1, dans lequel l'étape d'évaluation comporte l'estimation (208) d'un risque de séquestration de sinus ; ou dans lequel l'étape d'évaluation comporte l'estimation (210) d'un risque d'obstruction d'accès à l'artère coronaire.

5. Procédé selon la revendication 1, dans lequel la combinaison valve-dans-valve définit une néo-jupe, et en outre dans lequel les mesures de référence prédéterminées comprennent une hauteur de la néo-jupe et un diamètre de la combinaison valve-dans-valve.

6. Procédé selon la revendication 5, dans lequel la néo-jupe est générée par des feuillets de la valve aortique bioprothétique connue coincés entre une structure d'endoprothèse de la valve aortique bioprothétique et une structure d'endoprothèse de la valve aortique transcathéter connue.

7. Procédé selon la revendication 6, dans lequel les feuillets de la valve aortique bioprothétique connue sont soit entièrement épinglés, soit partiellement épinglés entre les structures d'endoprothèse.

8. Procédé selon la revendication 6, dans lequel la hauteur de la néo-jupe est définie par une distance entre une extrémité d'entrée de la structure d'endoprothèse de la valve aortique bioprothétique connue et un bord épinglé des feuillets ; ou dans lequel le diamètre est obtenu au niveau d'un plan d'un bord épinglé de la néo-jupe.

9. Procédé selon la revendication 1, dans lequel la première valve aortique bioprothétique est une valve aortique bioprothétique préalablement implantée, et les mesures anatomiques comportent une distance entre chaque ostium d'artère coronaire native et une extrémité d'entrée de la valve aortique bioprothétique préalablement implantée.

10. Procédé selon la revendication 9, dans lequel les mesures anatomiques comportent en outre une distance entre l'extrémité d'entrée et une jonction sinotubulaire native.

11. Procédé selon la revendication 9, dans lequel les mesures anatomiques comportent en outre un paramètre indicatif d'un espacement entre la valve aortique bioprothétique préalablement implantéeet une paroi aortique native ; et éventuellement dans lequel le paramètre est choisi dans le groupe constitué par une distance, une aire résiduelle et un volume résiduel.

12. Procédé selon la revendication 1, dans lequel l'étape d'examen comporte la comparaison d'une valeur de hauteur de néo-jupe des mesures de référence prédéterminées avec une valeur de hauteur d'ostium d'artère coronaire des mesures anatomiques ; ou dans lequel l'étape d'examen comprend la comparaison d'une valeur de hauteur de néo-jupe des mesures de référence prédéterminées avec une hauteur de jonction sinotubulaire des mesures anatomiques.

13. Procédé selon la revendication 1, dans lequel l'étape d'examen comporte l'estimation d'un paramètre indicatif d'un espacement entre la première valve aortique bioprothétique et l'anatomie native au niveau d'une jonction sinotubulaire native ; et éventuellement dans lequel le paramètre est choisi dans le groupe constitué par une distance, une aire résiduelle et un volume résiduel.

14. Procédé selon la revendication 1, dans lequel l'étape d'examen comprend l'évaluation d'un paramètre indicatif d'un espacement entre la première valve aortique bioprothétique et l'anatomie native à un niveau correspondant à une valeur de hauteur de néo-jupe des mesures de référence prédéterminées ; et éventuellement dans lequel le paramètre est choisi dans le groupe constitué par une distance, une aire résiduelle et un volume résiduel.

15. Procédé selon la revendication 1, dans lequel les mesures de référence prédéterminées d'une combinaison valve dans valve sont obtenues par le biais d'essai au banc ; ou dans lequel les mesures de référence prédéterminées sont obtenues à partir d'une bibliothèque, et en outre dans lequel la bibliothèque conserve des mesures de référence prédéterminées pour une pluralité de combinaisons valve dans valve différentes.
